# EUROPEAN PATENT SPECIFICATION

(11) **EP 1 422 195 B1**
(45) Date of publication and mention of the grant of the patent: **10.12.2008**
(21) Application number: 02705293.5
(22) Date of filing: 18.03.2002
(51) Int. Cl.: A61L 9/01, A61L 9/014, A61L 2/232, C02F 1/72, C01B 13/02, C01B 15/01

(54) **ACTIVE OXYGEN GENERATING MATERIAL**
AKTIVSAUERSTOFF ERZEUGENDES MATERIAL
MATIERE GENERATRICE D'OXYGENE ACTIF

(30) Priority: 26.06.2001 JP 2001192376
(43) Date of publication of application: 26.05.2004
(73) Proprietor: National Institute of Advanced Industrial Science and Technology, Tokyo 100-8921 (JP)
(72) Inventor: NONAMI, Toru, AIST Chubu, Nagoya-shi, Aichi 463-8560 (JP)
(74) Representative: Ebner von Eschenbach, Jennifer
(86) International application number: PCT/JP2002/002545
(87) International publication number: WO 2003/000587

(56) References cited:
- EP-A- 0 782 970
- WO-A-01/14257
- JP-A- 1 060 639
- JP-A- 10 316 403
- JP-A- 11 158 675
- JP-A- 11 241 021
- US-A- 5 755 969

## Description

### BACKGROUND OF THE INVENTION

### 1. Field of the Invention

The present invention relates to an active oxygen generating material comprising a polyaniline catalyst powder, and more particularly to an active oxygen generating agent that is capable, for example, of destroying microorganisms, bacteria, and other organisms living in the water, air, or the like, that generates active oxygen useful in adsorbing and decomposing harmful chemical substances, and that contains polyaniline coated with calcium phosphate; to a manufacturing method thereof; and to a method for generating active oxygen with the aid of this active oxygen generating agent.

### 2. Description of the Related Art

In conventional practice, known methods of destroying microorganisms, bacteria, and other organisms living in the water, air, or the like, and decomposing harmful chemical substances and the like, have included, for example, drug methods for treating with drugs, methods of destroying bacteria by electrolysis, ultraviolet irradiation methods, photocatalytic methods, active oxygen generating methods based on active oxygen generating agents, and the like, and recently active oxygen generating methods that do not require the use of large equipment, expensive drugs, or the like have become the focus of attention among these methods. Known examples of these methods include 1) a method for dissolving hydrogen peroxide using drugs or the like to generate active oxygen, 2) a method for adding chlorophyll to an aqueous solution and irradiating it with light to generate superoxide, which is one type of active oxygen, 3) a method for bringing a liquid that contains dissolved oxygen in contact with an active oxygen generating agent containing polyaniline to generate active oxygen (Japanese Patent Application Laid-Open No. H9-175801 & EP-A-0 782 970), and the like.

The polyaniline-containing active oxygen generating agent can readily generate active oxygen without the need for specific equipment or procedures by merely coming into contact with a liquid that contains dissolved oxygen.

However, there are problems with polyaniline in that it loses activity if not dried and cannot be continuously used because it is incapable of adsorbing matter, and another drawback is that it is difficult to remove bacteria or the like. There have also been drawbacks in that when this material is mixed with a resin or plastic, the medium itself deteriorates.

Amidst these circumstances and in view of the above-mentioned prior art, the inventor of the present invention, as a result of various studies with the intention to develop a new active oxygen generating agent capable of reliably solving the above-mentioned problems of the prior art, has discovered that the desired object can be achieved and have completed the present invention by using polyaniline compounded or mixed with calcium phosphate as an active ingredient.

### SUMMARY OF THE INVENTION

The present invention provides an active oxygen generating material and a manufacturing method thereof.

The present invention relates to an active oxygen generating agent wherein a porous calcium phosphate coating is formed on at least part of the surface of polyaniline particles, and the polyaniline compounded or mixed with the calcium phosphate is contained as an active ingredient; a method for manufacturing an active oxygen generating agent component wherein calcium phosphate is precipitated onto the surface of polyaniline by immersing the polyaniline in a pseudo-fluid comprising calcium ions and phosphate ions; a method for generating active oxygen by bringing a liquid that contains dissolved oxygen in contact with the active oxygen generating agent; and a composition for environmental clarification.

### DISCLOSURE OF THE INVENTION

Specifically, as a result of much earnest research intended to achieve the above-mentioned objectives, the inventor has discovered that a durable calcium phosphate coating can be obtained by dispersing polyaniline in advance in an aqueous slurry comprising metal ions or an anionic surfactant and then forming a coating of calcium phosphate on all or part of the surface of the polyaniline microparticles; and also that because this coating is porous and has properties of adsorbing microorganisms and the like, there is no loss of polyaniline functions, and when the coating is used while supported on an organic polymer or other such medium, the durability of the medium improves dramatically.

The present invention provides a polyaniline catalyst powder for environmental clarification consisting of powder wherein a porous calcium phosphate coating layer is formed on at least part of the surface of the polyaniline particles.

In view of the problems with the prior art, an object of the present invention is to provide a powder for environmental clarification that can efficiently, economically, and safely perform environmental clarification, such as the removal of bad odors, the decomposition and removal of harmful airborne substances or contaminants, wastewater treatments or purification treatments, and antibacterial and mildewproofing treatments; and particularly that displays excellent polyaniline catalyst action even in terms of durability without causing degradation of a medium when used while supported by kneading or embedding into organic fiber, plastic, or another such organic polymer medium.

Another object of the present invention is to provide a method for manufacturing such a polyaniline catalyst powder for environmental clarification.

Yet another object of the present invention is to provide an organic polymer composition containing such a polyaniline catalyst powder for environmental clarification.

Still another object of the present invention is to provide molded articles molded from the organic polymer composition, and to provide a method for manufacturing those molded articles.

The present invention for solving the above-mentioned problems comprises the following technical means.
(1) An active oxygen generating agent, which comprises, as an active ingredient, polyaniline compounded or mixed with calcium phosphate obtainable by forming a porous calcium phosphate coating on at least part of the surface of polyaniline particles.
(2) The active oxygen generating agent according to (1), wherein the polyaniline is polyaniline with calcium phosphate crystals precipitated on the surface thereof.
(3) The active oxygen generating agent according to (1)or (2), wherein the mixture ratio of calcium phosphate and polyaniline is 0.001 to 200% of calcium phosphate in relation to polyaniline.
(4) The active oxygen generating agent according to (2), wherein the calcium phosphate crystals are apatite, octacalcium phosphate (OCP), and/or tetracalcium phosphate (TCP) having a tabular or columnar configuration
(5) The active oxygen generating agent according to (2), wherein the calcium phosphate crystals cover part of the polyaniline surface, and part of the polyaniline surface is exposed.
(6) The active oxygen generating agent according to (1), wherein the calcium phosphate covers 0.001 to 99% of the surface area of the polyaniline.
(7) An active oxygen generating agent in thin film form, which comprises polyaniline coated with calcium phosphate obtainable by forming a composition consisting of an organic polymer and polyaniline compounded or mixed with calcium phosphate defined in (1) into a thin film, said polyaniline compounded or mixed with calcium phosphate accounts for 0.01 to 95% of the surface of the thin film.
(8) The active oxygen generating agent according to (1), wherein the particle diameter of the polyaniline is 5 nm to 20 micrometer.
(9) The active oxygen generating agent according to (1), wherein the polyaniline is reduced type polyaniline.
(10) The active oxygen generating agent according to (1), wherein the agent containes a ceramic catalyst.
(11) The active oxygen generating agent according to (10), wherein the ceramic catalyst is photocatalytic titanium dioxide.
(12) The active oxygen generating agent according to (1), wherein the agent has configuration of powder, granules, or a thin film.
(13) A method for manufacturing an active oxygen generating agent according to (1), which comprises immersing the polyaniline in a pseudo-fluid containing calcium ions and phosphate ions to precipitate calcium phosphate onto the surface of polyaniline.
(14) A method for generating active oxygen, which comprises contacting a liquid that contains dissolved oxygen with an active oxygen generating agent coated with the calcium phosphate defined in any one of (1) through (12).
(15) A composition for environmental clarification having active oxygen generating action, which comprises, as an active ingredient, the polyaniline compounded or mixed with calcium phosphate defined in (1).

Next, the present invention will be described in further detail.

The polyaniline used in the present invention may be either oxidized or reduced. In this polyaniline, alkyl groups or other such groups may be substituted on the aromatic rings. It is possible to use either those doped with sulfuric acid, hydrochloric acid, or another such protonic acid or Lewis acid, or those de-doped. Also, the degree of polymerization of the polyaniline is not limited.

This polyaniline can be manufactured by chemical synthesis methods using an oxidant, electrochemical synthesis methods, or other such conventionally known methods. A persulfate, Lewis acid, or other such known oxidant can be used as the oxidant in the above-mentioned chemical synthesis methods.

In common chemical synthesis methods, doped polyaniline (AA) is obtained because aniline is dissolved in an acidic aqueous solution, an oxidant is added, and polymerization is performed. De-doped polyaniline (AB) is obtained when the product is neutralized using an alkali. These two types of polyaniline (AA, AB) are referred to as oxidized polyaniline to distinguish them from the reduced type described hereinbelow. AB is also referred to as emeraldine, of which AA is a salt. These oxidized polyanilines (AA, AB) are easily reduced by electrochemical reduction or hydrazine or another such weak reducing agent, resulting in the structure BA or BB. Normally an oxidized polyaniline (AA, AB) and a reduced polyaniline (BA, BB) are said to both be present in the polyaniline at about 50% each. The above-mentioned BB is also referred to as leuco emeraldine, of which BA is a salt. Reduced polyaniline (BA or BB) is automatically oxidized to polyaniline when allowed to stand in the air. Polyaniline does not change when allowed to stand in the air, but when further oxidized, the ratio of x/y in the oxidized polyaniline (AA, AB) decreases; specifically, polyaniline with less pronounced reduction and enhanced oxidation is generated, and when even further oxidized, pernigranilin (CB) or a salt thereof (CA) is obtained. The inventors have confirmed that this CB or CA also generates active oxygen. Also, the repeating unit n usually number 2 to 1000, and preferably 10 to 500.

In the present invention, "active oxygen" refers to oxygen that has much higher activity than normal oxygen and that is apt to undergo chemical reactions, and specifically means singlet oxygen, the superoxide anion radical (·O²⁻), hydroxy radical (·OH), perhydroxy radical (·OOH), and hydrogen peroxide.

In the present invention, a polyaniline catalyst powder for environmental clarification is manufactured by dispersing polyaniline microparticles in an aqueous slurry containing, for example, metal ions or an anionic surfactant and then forming a coating of porous calcium phosphate on at least part of the surface of the polyaniline microparticles.

Also in the present invention, a polymer composition is produced by mixing the above-mentioned polyaniline catalyst powder for environmental clarification with an organic polymer in an amount of 0.01 to 80% by weight, based on the total weight of the composition.

Also in the present invention, molded polymer articles having environmental clarification functions are produced by molding the above-mentioned polymer composition.

Furthermore in the present invention, molded polymer articles having environmental clarification functions are manufactured by kneading the above-mentioned polymer composition in an extruder and extrusion molding the resulting material with the extruder.

The above-mentioned polyaniline catalyst powder for environmental clarification is characterized by the presence of polyaniline microparticles and a coating layer of porous calcium phosphate formed on at least part of the surface thereof.

In the polyaniline microparticles used to manufacture such polyaniline catalyst powder for environmental clarification preferably, the average particle diameter of primarily particles is 0.001 to 0.2 micrometer. When the average particle diameter is less than 0.001 micrometer, efficient production is difficult to achieve and impractical to accomplish. Conversely, when the average particle diameter exceeds 0.2 micrometer, polyaniline performs much more poorly as a catalyst. A more preferable average particle diameter for the primary particles is 0.005 to 20 micrometer.

A coating of porous calcium phosphate is formed on at least part of the surface of the polyaniline microparticles. Methods in which a pseudo-fluid comprising at least calcium ions and phosphate ions is brought into contact with polyaniline microparticles, and calcium phosphate is precipitated on the surface of the microparticles are normally utilized to form the porous calcium phosphate coating.

The pseudo-fluid used here is a working fluid that yields calcium phosphate Ca₃(PO₄)₂ or any other calcium phosphate compound shown by various rational formulas, and this working fluid is produced by dissolving, for example, NaCl, NaHCO₃, KCl, K₂HPO₄·3H₂O, MgCl_{2.6}H₂O, CaCl₂, Na₂SO₄, NaF, or the like in water. It is also preferable to adjust the pH to 7 to 8, particularly 7.4, with HCl, (CH₂OH)₃CNH₂, or the like. The Ca²⁺ ion concentration of the pseudo-fluid used herein is preferably 0.1 to 50 mM, and the phosphate ion concentration is preferably 0.1 to 20 mM. Precipitation of the calcium phosphate requires much time if the Ca²⁺ ion and phosphate ion concentrations are diluted any further, and, conversely, if the concentrations are any higher, production occurs too rapidly and it becomes difficult to control the degree of porosity and film thickness.

The resulting porous calcium phosphate may be any kind of porous calcium phosphate consisting of phosphate ions and calcium ions. These are compounds with Ca₉(PO₄)₆ as basic units, and specific examples include calcium tertiary phosphate Ca₃(PO₄)₂, hydroxyapatite Ca₁₀(PO₄)₆(OH)₂. calcium secondary phosphate·dihydrate CaHPO₄·2H₂O, octacalcium phosphate Ca₈H₇(PO₄)₆·5H₂O, tetracalcium phosphate Ca₄O(PO₄)₂, and the like. Of these, apatite is preferable, and amorphous apatite is particularly preferable. There is no particular distinction made for the configuration of the coating, but it is preferable to coat the polyaniline surface as uniformly as possible. In this case the calcium phosphate crystals cover part of the surface of the polyaniline, and it is preferable for part of the polyaniline surface to be exposed, with the calcium phosphate covering 0.001 to 99% of the polyaniline surface.

The amount of porous calcium phosphate precipitated on the surface of the polyaniline microparticles is preferably about 0.01 to 200% by weight based on the weight of the polyaniline. When the amount of porous calcium phosphate is insufficient, the amount in which harmful substances, malodorous substances, and other components are adsorbed by the porous calcium phosphate is reduced, and therefore the decomposition and removal efficiency of these substances is reduced, degradation of the medium that supports the polyaniline catalyst powder is brought about, and the durability of the polyaniline catalyst function is reduced. When the amount is too large, it is difficult for the polyaniline function to be adequately demonstrated. The polyaniline slurry that has been coated on the porous calcium phosphate is ultimately dried to yield a polyaniline catalyst powder for environmental clarification.

Since the calcium phosphate film coating on the surface of the microparticles is porous, the polyaniline catalyst powder for environmental clarification of the present invention has sections in fine pores where the polyaniline surface remains exposed without being covered, and environment clarification catalytic action can be provided by the polyaniline in these sections. Specifically, due to the oxidation-reduction action of electrons and holes produced by the powder, it is easy to decompose and remove odoriferous organic substances adsorbed on the calcium phosphate, airborne harmful substances, organic solvents dissolved in the water, agricultural chemicals, and other such organic compounds that contaminate the environment.

Since the durable porous calcium phosphate film obtained by the above-mentioned methods prevents direct contact between the polyaniline and the medium, the fiber and plastic themselves are less likely to be decomposed or otherwise degraded, and the effects of the polyaniline catalyst can be sustained for long periods of time even when the polyaniline catalyst powder for environmental clarification is used after being added to organic fiber, plastic, or another such organic polymer medium by kneading or the like.

Furthermore, since the porous calcium phosphate film has the properties of adsorbing bacteria, proteins, amino acids, airborne or waterborne bacteria, viruses, odoriferous organic substances, nitrogen oxides, and the like, the adsorbed bacteria and the like can be reliably and efficiently destroyed or decomposed by the strong oxidative power of the polyaniline. The proteins, amino acids, bacteria, viruses, and the like adsorbed by the porous calcium phosphate film can then be quickly and continuously decomposed and removed.

Particularly, when the above-mentioned polyaniline catalyst powder for environmental clarification is used after being kneaded or embedded into organic fiber, plastic, or another such organic polymer medium, it is possible to adsorb and continuously decompose and remove odor, NOx, or other such airborne organic substances, organic solvents dissolved in the water, agricultural chemicals, and other such organic compounds that contaminate the environment, without any decomposition of the organic fiber or plastic.

The environment clarification powder of the present invention can be applied to organic fiber and plastic molded articles consisting of polyethylene, nylon, polyvinyl chloride, polyvinylidene chloride, polyester, polypropylene, polyethylene oxide, polyethylene glycol, polyethylene terephthalate, silicon resin, polyvinyl alcohol, vinyl acetal resin, polyacetate, ABS resin, epoxy resin, vinyl acetate resin, cellulose, cellulose derivative, polyamide, polyurethane, polycarbonate, polystyrene, urea resin, fluororesin, polyvinylidene fluoride, phenol resin, celluloid, chitin, starch sheet, and all other types of organic polymers; and molded polymer articles having environment clarification functions can be obtained by molding compositions consisting of the above-mentioned polyaniline catalyst powders and organic polymers. Generally, the powder is supported with the aid of kneading, embedding, or other such means implemented in steps in which organic fiber or plastic molded articles are manufactured. Particularly, it is preferable to obtain molded polymer articles having environment clarification functions by mixing and extrusion molding an organic polymer and powder using an extruder. The concentration of powder in the polymer composition consisting of powder and an organic polymer is normally 0.01 to 95% by weight, and preferably 1 to 50% by weight, based on the weight of the polymer composition.

In the active oxygen generating agent of the present invention, a ceramic catalyst such as a titanium dioxide photocatalyst, activated carbon, zeolite, or another such powder may be blended in addition to the above-mentioned polyaniline. Possible examples of other additives include carbon powder and conductive fiber. When an additive is used in this manner, the content percentage of the polyaniline is normally 1 to 100% by weight, and preferably 10 to 70% by weight in relation to the entire active oxygen generating agent. The active oxygen generating agent of the present invention is not limited as to its configuration, and may be in the form of powder or pellets. The polyaniline may be affixed to the surface of the conductive fiber, such as carbon fiber, in the form of a film. The titanium dioxide is preferably one coated with apatite. When polyaniline is soaked in a pseudo-fluid, titanium dioxide is soaked at the same time and similarly aged in order to allow the titanium dioxide to be compounded. Calcium phosphate is simultaneously precipitated on the surface of polyaniline and titanium dioxide, yielding a composite powder.

Next, the method for generating active oxygen of the present invention will be described. This method involves generating active oxygen by bringing a liquid that contains dissolved oxygen in contact with the above-mentioned active oxygen generating agent of the present invention.

The liquid used in the present invention is usually one that is the object of sterilization, generally water. This water is not particularly limited as long as oxygen is dissolved therein, and possible examples include tap water, well water, spring water, purified water, and the like. It may also be physiological saline, a buffer solution, and other types of liquid containing electrolytes and other soluble substances. Examples of liquids other than water include dimethyl sulfoxide, dimethyl formamide, acetonitrile, pyridine, and the like. These liquids normally contain dissolved oxygen from the air. The dissolved percentage is normally 5 to 500 ppm.

The method for bringing the above-mentioned liquid and the active oxygen generating agent of the present invention into contact with each other is not particularly limited. Possible examples include methods of introducing the active oxygen generating agent of the present invention in powder or pellet form to the above-mentioned liquid and stirring, and methods of supporting the active oxygen generating agent of the present invention on porous material and passing the above-mentioned liquid therethrough to continuously generate active oxygen. Additional methods include supporting the active oxygen generating agent of the present invention on conductive woven fabric or felt material and passing the above-mentioned liquid therethrough while applying a reduction potential to continuously generate active oxygen.

Examples of means for generating active oxygen in greater quantities include methods of supplying air or oxygen-rich gas to the above-mentioned liquid, methods of repeatedly maintaining contact with polyaniline, and methods of maintaining contact while subjecting oxidized polyaniline to reduction. The active oxygen generating agent of the present invention is also cost effective because after it is used once, the active oxygen generating functions can be restored and used repeatedly upon filtering and drying.

The present invention provides a novel active oxygen generating agent coated with calcium phosphate whereby it is possible to easily generate active oxygen at low cost without any problems of environmental pollution, and also provides an active oxygen generating method using the same.

With the active oxygen generating agent of the present invention, it is important to implement a structure containing polyaniline coated with calcium phosphate.

It is possible due to the present invention to solve problems of environmental pollution, problems caused by complex operations, cost-related problems, and other such conventional problems.

The active oxygen generating agent of the present invention may consist solely of polyaniline coated with calcium phosphate.

In addition, the active oxygen generating agent of the present invention, after being used once, can be restored to its initial activity and reused by filtration and drying. The active oxygen generating agent of the present invention presents little danger that environmental pollution will occur. Therefore, if the active oxygen generating agent of the present invention is used, it is possible to solve problems of environmental pollution, problems caused by complex operations, cost-related problems, and other such conventional problems.

### BRIEF DESCRIPTION OF THE DRAWINGS

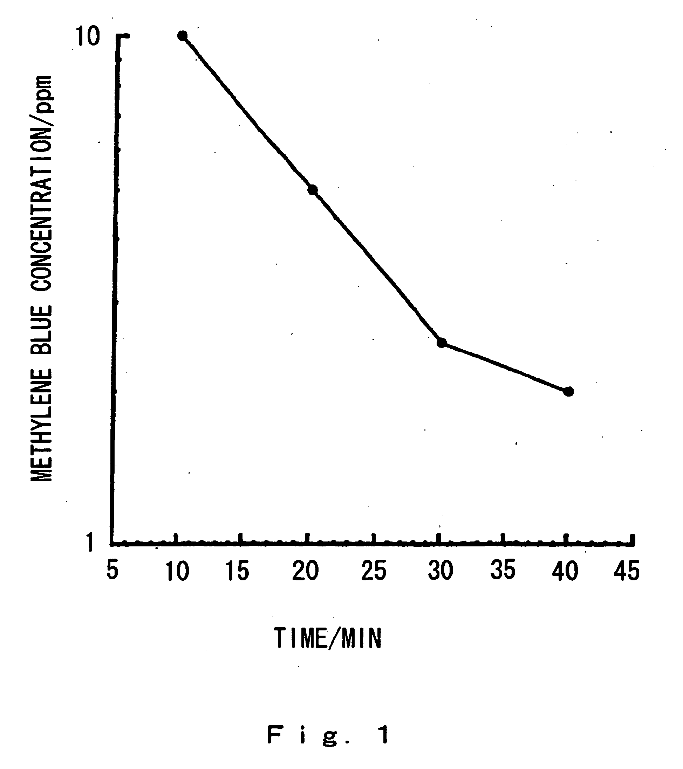

FIG. 1 shows the decomposition of a methylene blue solution (10 ppm) based on the active oxygen generating agent of the present invention;
FIG. 2 shows the decomposition of a methylene blue solution (10 ppm) based on the active oxygen generating agent of the present invention; and
FIG. 3 shows the decomposition of a acetaldehyde solution based on the active oxygen generating agent of the present invention.

### DESCRIPTION OF THE PREFERRED EMBODIMENTS

Next, the present invention will be described in detail based on examples, but the present invention is in no way limited by these examples.

### Example 1

3 g of polyaniline ultrafine particles (made by Nitto Denko, average particle diameter of primary particles: 5 micron) were added to and dispersed in 1 L of purified water. In addition, NaCl, NaHPO₄, KH₂PO₄, KCl, MgCl₂·6H₂O, and CaCl₂ were added to purified water, and 300 mL of a pseudo-fluid was produced such that after mixing with a slurry, there were 139 mM of Na⁺, 2.8 mM of K⁺, 1.8 mM of Ca²⁺, 0.5 mM of Mg²⁺,144 mM of Cl⁻, and 1.1 mM of HPO₄⁻250 cc of a polyaniline slurry obtained by the above-mentioned methods were mixed with 300 mL of a pseudo-fluid, and the resulting material was kept for 24 hours while holding the temperature at 40°C. After 4 hours, upon analyzing the precipitated amount of calcium phosphate, it was confirmed that about 90% of the finally precipitated amount had precipitated. The slurry was then dried to obtain 3 g of a photocatalytic powder for environmental clarification. Upon analyzing the precipitate, it was found to be apatite-containing calcium phosphate.

This powder generated hydrogen peroxide in purified water. When 2 to 6 wt% of this powder was mixed with polyethylene, there were no changes in color or other such changes even after one month. It was found that the powder was capable of adsorbing and decomposing E. coli. The data is shown in Table 1.

**TABLE 1**

| | Bacterial Count, No./mL | Hydrogen Peroxide Concentration/ppm |
|---|---|---|
| Control | 8300 | 0 |
| 2 wt% added over 20 min | 500 | 2 |
| 2 wt% added over 180 min | 10 or less | 5 |
| 4 wt% added over 20 min | 10 or less | 10 |
| 4 wt% added over 120 min | 10 or less | 20 |
| 6 wt% added over 20 min | 10 or less | 20 |
| 6 wt% added over 120 min | 10 or less | 30 |

### Example 2

The slurry in Example 1 was filtered, washed, mixed with 5% of an organic binder, and made into paint. A metal substrate was coated with the resulting material to produce a thin film of about 1 micron.

It was clear that this thin film was capable of generating active oxygen in a liquid and decomposing organic matter. For example, the substrate decomposed when placed in 3 mL of a methylene blue (10 ppm) solution. The data is shown in Table 1.

### Example 3

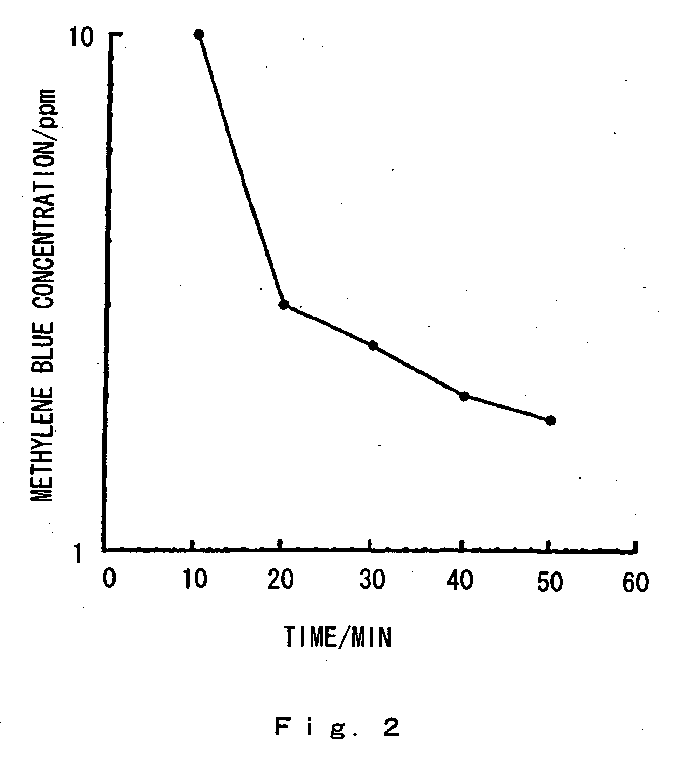

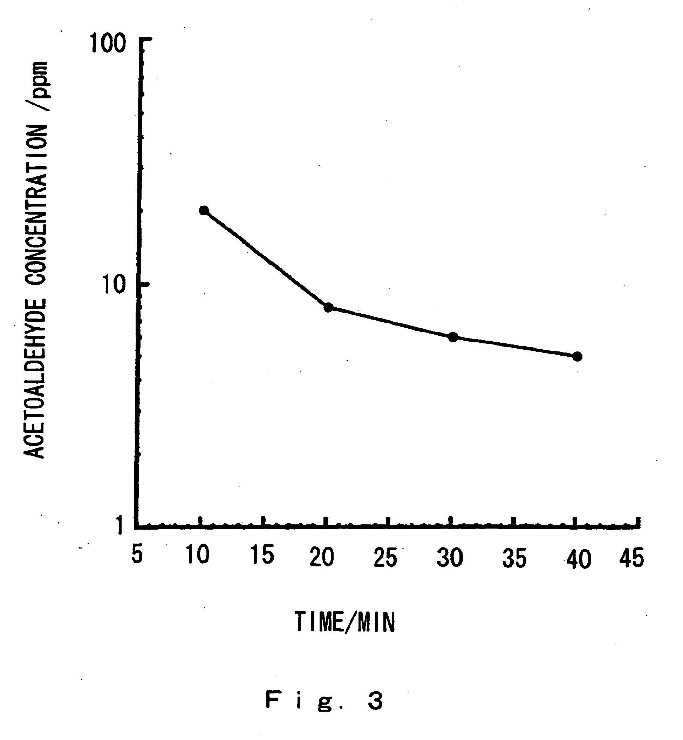

1 g of titanium dioxide (made by Aldrich, high grade reagent) was dispersed together with polyaniline in purified water, but otherwise the treatment was the same as Example 1. As a result, calcium phosphate precipitated on the surface of the polyaniline and titanium dioxide. This composite powder generated hydrogen peroxide in water and also generated active oxygen when irradiated with light. It was clear that the resulting complex was capable of adsorbing and decomposing bacteria, dyes, and the like in water. The data is shown in Figs. 2 and 3.

As described above, the present invention relates to an active oxygen generating agent characterized in containing, as an active ingredient, polyaniline compounded or mixed with calcium phosphate by forming a porous calcium phosphate coating on at least part of the surface of polyaniline particles, and the following effects are achieved by the present invention.
(1) A novel active oxygen generating agent coated with calcium phosphate can be provided.
(2) Active oxygen can easily be generated at low cost by simple operations.
(3) Microorganisms, bacteria, and the like can be destroyed, and harmful chemical substances can be adsorbed and decomposed by using the above-mentioned active oxygen generating agent.
(4) The agent can be mixed and kneaded with organic resins or paint.
(5) The hydrophilicity of polyaniline can be improved by coating with calcium phosphate.
(6) Functions for adsorbing substances can be added, making continuous use possible.
(7) Removal of bacteria and the like is easy.

## Claims

1. An active oxygen generating agent, which comprises, as an active ingredient, polyaniline compounded or mixed with calcium phosphate obtainable by forming a porous calcium phosphate coating on at least part of the surface of polyaniline particles.

2. The active oxygen generating agent according to claim 1, wherein the polyaniline comprises calcium phosphate crystals precipitated on the surface thereof.

3. The active oxygen generating agent according to claim 1 or 2, wherein the mixture ratio of calcium phosphate and polyaniline is 0.001 to 200% of calcium phosphate in relation to polyaniline.

4. The active oxygen generating agent according to claim 2, wherein the calcium phosphate crystals are apatite, octacalcium phosphate (OCP), and/or tetracalcium phosphate (TCP) having a tabular or columnar configuration.

5. The active oxygen generating agent according to claim 2, wherein the calcium phosphate crystals cover part of the polyaniline surface, and part of the polyaniline surface is exposed.

6. The active oxygen generating agent according to claim 1, wherein the calcium phosphate covers 0.001 to 99% of the surface area of the polyaniline.

7. An active oxygen generating agent in thin film form, which comprises polyaniline coated with calcium phosphate obtainable by forming a composition consisting of an organic polymer and polyaniline compounded or mixed with calcium phosphate defined in claim 1 into a thin film, said polyaniline compounded or mixed with calcium phosphate accounts for 0.01 to 95% of the surface of the thin film.

8. The active oxygen generating agent according to claim 1, wherein the particle diameter of the polyaniline is 5 nm to 20 micrometer.

9. The active oxygen generating agent according to claim 1, wherein the polyaniline is reduced type polyaniline.

10. The active oxygen generating agent according to claim 1, wherein the agent containes a ceramic catalyst.

11. The active oxygen generating agent according to claim 10, wherein the ceramic catalyst is photocatalytic titanium dioxide.

12. The active oxygen generating agent according to claim 1, wherein the agent has configuration of powder, granules, or a thin film.

13. A method for manufacturing an active oxygen generating agent according to claim 1, which comprises immersing the polyaniline in a fluid containing calcium ions and phosphate ions to precipitate calcium phosphate onto the surface of polyaniline.

14. A method for generating active oxygen, which comprises contacting a liquid that contains dissolved oxygen with the active oxygen generating agent defined in any one of claims 1 through 12.

15. A composition for environmental clarification having active oxygen generating action, which comprises, as an active ingredient, the polyaniline compounded or mixed with calcium phosphate defined in claim 1.

## Patentansprüche

1. Aktiven Sauerstoff erzeugendes Mittel, wobei das Mittel als einen aktiven Bestandteil Polyanilin aufweist, das compoundiert ist oder gemischt ist mit Calciumphosphat, das erhalten werden kann, indem eine poröse Calciumphosphat-Beschichtung auf mindestens einem Teil der Oberfläche der Polyanilin-Partikel erzeugt wird.

2. Aktiven Sauerstoff erzeugendes Mittel nach Anspruch 1, wobei das Polyanilin Calciumphosphat-Kristalle aufweist, die auf deren Oberfläche abgeschieden sind.

3. Aktiven Sauerstoff erzeugendes Mittel nach Anspruch 1 oder 2, wobei das Mischungsverhältnis von Calciumphosphat und Polyanilin 0,001% bis 200% Calciumphosphat in Bezug auf Polyanilin beträgt.

4. Aktiven Sauerstoff erzeugendes Mittel nach Anspruch 2, wobei die Calciumphosphat-Kristalle Apatit sind, Oktacalciumphophat (OCP) und/oder Tetracalciumphosphat (TCP), die eine tafelförmige oder stengelförmige Konfiguration haben.

5. Aktiven Sauerstoff erzeugendes Mittel nach Anspruch 2, wobei die Calciumphosphat-Kristalle einen Teil der Polyanilin-Oberfläche bedecken und ein Teil der Polyanilin-Oberfläche exponiert ist.

6. Aktiven Sauerstoff erzeugendes Mittel nach Anspruch 1, wobei das Calciumphosphat 0,001 % bis 99% der Oberfläche des Polyanilin bedeckt.

7. Aktiven Sauerstoff erzeugendes Mittel in Form eines Dünnschichtfilms, welches mit Calciumphosphat beschichtetes Polyanilin aufweist, das erhalten werden kann, indem eine nach Anspruch 1 aus einem organischen Polymer und Polyanilin bestehende Zusammensetzung, compoundiert oder gemischt mit Calciumphosphat zu einem Dünnschichtfilm erzeugt wird, wobei das mit Calciumphosphat compoundierte oder gemischte Polyanilin 0,01% bis 95% der Oberfläche des Dünnschichtfilms ausmacht.

8. Aktiven Sauerstoff erzeugendes Mittel nach Anspruch 1, wobei der Partikeldurchmesser des Polyanilins 5 Nanometer bis 20 Mikrometer beträgt.

9. Aktiven Sauerstoff erzeugendes Mittel nach Anspruch 1, wobei das Polyanilin vom Typ des reduzierten Polyanilins ist.

10. Aktiven Sauerstoff erzeugendes Mittel nach Anspruch 1, wobei das Mittel einen Keramik-Katalysator enthält.

11. Aktiven Sauerstoff erzeugendes Mittel nach Anspruch 10, wobei der Keramik-Katalysator photokatalytisches Titandioxid ist

12. Aktiven Sauerstoff erzeugendes Mittel nach Anspruch 1, wobei das Mittel die Konfiguration von Pulver, Granalien oder die eines Dünnschichtfilms hat.

13. Verfahren zum Herstellen eines aktiven Sauerstoff erzeugenden Mittels nach Anspruch 1, wobei das Verfahren das Eintauchen des Polyanilins in eine Flüssigkeit umfasst. die Calcium-lonen und Phosphat-Ionen enthält, um auf der Oberfläche des Polyanilins Calciumphosphat abzuscheiden.

14. Verfahren zum Erzeugen von aktivem Sauerstoff, wobei das Verfahren das Kontaktieren einer aufgelösten Sauerstoff enthaltenden Flüssigkeit mit dem aktiven Sauerstoff erzeugenden Mittel nach einem der Ansprüche 1 bis 12 umfasst.

15. Zusammensetzung zur Umgebungsreinigung mit aktiven Sauerstoff erzeugender Wirkung, wobei die Zusammensetzung als einen aktiven Bestandteil das mit Calciumphosphat compoundierte oder gemischte Polyanilin nach Anspruch 1 aufweist.

## Revendications

1. Agent générant de l'oxygène actif, qui comprend, en tant qu'ingrédient actif, de la poly(aniline) combinée ou mélangée à du phosphate de calcium pouvant être obtenu en formant un revêtement de phosphate de calcium poreux sur au moins une partie de la surface de particules de poly(aniline).

2. Agent générant de l'oxygène actif selon la revendication 1, dans lequel la poly(aniline) comprend des cristaux de phosphate de calcium précipités sur sa surface.

3. Agent générant de l'oxygène actif selon la revendication 1 ou 2, dans lequel le rapport de mélange du phosphate de calcium et de la poly(aniline) est de 0,001 à 200 % de phosphate de calcium par rapport à la poly(aniline).

4. Agent générant de l'oxygène actif selon la revendication 2, dans lequel les cristaux de phosphate de calcium sont de l'apatite, du phosphate d'octacalcium (OCP) et/ou du phosphate de tétracalcium (TCP) ayant une configuration tabulaire ou colonnaire.

5. Agent générant de l'oxygène actif selon la revendication 2, dans lequel les cristaux de phosphate de calcium recouvrent une partie de la surface en poly(aniline) et une partie de la surface en poly(aniline) est exposée.

6. Agent générant de l'oxygène actif selon la revendication 1, dans lequel le phosphate de calcium recouvre 0,001 à 99 % de la surface de la poly(aniline).

7. Agent générant de l'oxygène actif sous forme de film mince, qui comprend de la poly(aniline) revêtue avec du phosphate de calcium pouvant être obtenu en formant une composition constituée d'un polymère organique et de poly(aniline) combinée ou mélangée à du phosphate de calcium défini dans la revendication 1 en un film mince, ladite poly(aniline) combinée ou mélangée à du phosphate de calcium représente 0,01 à 95 % de la surface du film mince.

8. Agent générant de l'oxygène actif selon la revendication 1, dans lequel le diamètre de particule de la poly(aniline) est de 5 nm à 20 micromètres.

9. Agent générant de l'oxygène actif selon la revendication 1, dans lequel la poly(aniline) est une poly(aniline) de type réduit.

10. Agent générant de l'oxygène actif selon la revendication 1, dans lequel l'agent contient un catalyseur céramique.

11. Agent générant de l'oxygène actif selon la revendication 10, dans lequel le catalyseur céramique est du dioxyde de titane photocatalytique.

12. Agent générant de l'oxygène actif selon la revendication 1, dans lequel l'agent présente une configuration de poudre, de granulés ou d'un film mince.

13. Procédé de fabrication d'un agent générant de l'oxygène actif selon la revendication 1, qui comprend l'immersion de la poly(aniline) dans un fluide contenant des ions calcium et des ions phosphate pour précipiter le phosphate de calcium sur la surface de la poly(aniline).

14. Procédé de génération d'oxygène actif, qui comprend la mise en contact d'un liquide qui contient de l'oxygène dissous avec l'agent générant de l'oxygène actif défini dans l'une quelconque des revendications 1 à 12.

15. Composition de clarification pour l'environnement ayant une action générant de l'oxygène actif, qui comprend, en tant qu'ingrédient actif, la poly(aniline) combinée ou mélangée à du phosphate de calcium défini dans la revendication 1.
